# DEMANDE DE BREVET EUROPEEN

(11) **EP 3 093 498 A1**
(43) Date de publication de la demande: **16.11.2016**
(21) Numéro de dépôt: 16305480.2
(22) Date de dépôt: 25.04.2016
(51) Int. Cl.: F04D 25/08, A61M 16/00, F04D 29/12

(54) **MICRO-SOUFFLANTE À ÉTANCHÉITÉ D'AXE-MOTEUR AMÉLIORÉE POUR APPAREIL D'ASSISTANCE RESPIRATOIRE**

(30) Priorité: 12.05.2015 FR 1554212
(71) Demandeur: Air Liquide Medical Systems, 92160 Antony (FR)
(72) Inventeur: GUIDUCCI, Hadrien, 75020 PARIS (FR)
(74) Mandataire: Pittis, Olivier

(57) **Abrégé**

L'invention porte sur une micro-soufflante (1) pour appareil d'assistance respiratoire comprenant un moteur électrique (9) comprenant un corps de moteur (2) et un arbre-moteur (3) rotatif de forme allongée, ledit arbre-moteur (3) étant maintenu ou guidé par au moins un dispositif à roulements (8) agencé dans ledit corps de moteur (2), ledit arbre-moteur (3) traversant la paroi supérieure (5) du corps de moteur (2) par un orifice (16) aménagé dans ladite paroi supérieure (5) et faisant saillie à l'extérieur dudit corps de moteur (2). Une pièce d'étanchéité (6) traversée par l'arbre-moteur (3) est agencée au niveau de l'orifice (16). Appareil d'assistance respiratoire équipé d'une telle micro-soufflante (1).

## Description

L'invention porte sur une micro-soufflante pour appareil d'assistance respiratoire dont l'étanchéité gazeuse a été améliorée et appareil d'assistance respiratoire équipé d'une telle micro-soufflante.

Afin d'assister certains patients dans leur fonction respiratoire, on utilise des appareils d'assistance respiratoire ou de ventilation assistée, aussi appelés ventilateurs médicaux, qui délivrent un gaz respiratoire à débit non nul et/ou à une pression supérieure à la pression atmosphérique (i.e. > 1 atm), typiquement de l'air. L'air envoyé au patient peut être additionné d'oxygène supplémentaire, notamment lorsque le patient doit recevoir, dans le cadre de son traitement, une proportion d'oxygène supérieure à 21% en volume.

Pour ce faire, on utilise des appareils d'assistance respiratoire mettant en oeuvre une micro-soufflante, parfois simplement appelée « soufflante » ou « turbine », servant à aspirer l'air ambiant et à le délivrer à une pression donnée aux patients. Les documents EP-A-2165078, EP-A-2102504 et WO-A-2012/139681 décrivent des micro-soufflantes et des appareils d'assistance respiratoire équipés de telles micro-soufflantes.

Classiquement, l'aspiration de l'air par la micro-soufflante se fait grâce à une (ou plusieurs) roue à ailettes agencée sur un arbre rotatif entraîné en rotation par un moteur électrique. La roue de la micro-soufflante est généralement agencée dans un compartiment aménagé entre une volute inférieure et une volute supérieure, fixées l'une à l'autre, définissant entre elles un volume creux au sein duquel peut tourner la roue, ladite roue étant prise en « sandwich » entre lesdites volutes.

Or, beaucoup de micro-soufflantes présentent un problème de durée de vie du moteur électrique lié notamment à une absence d'étanchéité gazeuse au niveau de l'arbre rotatif du moteur portant la roue à ailettes. En effet, la conception des micro-soufflantes actuelles ne permet pas de garantir une étanchéité entre le compartiment à roue rotative et l'intérieur du corps de moteur où se trouvent les roulements qui maintiennent ou guident l'axe-moteur rotatif.

Or, cette absence d'étanchéité est un problème important car il conduit à une usure prématurée du moteur.

Ainsi, elle conduit à des entrées d'air sous pression (> 1 atm) dans le corps du moteur où règne une plus basse pression (+/- 1 atm) du fait du différentiel de pression existant. Ces entrées d'air sous pression vont alors venir balayer les roulements et en chasser une partie du lubrifiant qui s'y trouve, typiquement de la graisse ou analogue, ce qui va progressivement conduire à un défaut de lubrification et inévitablement accélérer l'usure des composants en contact les uns avec les autres, en particulier des roulements.

De plus, lorsque la ventilation du patient se fait avec un flux d'air enrichi en oxygène, c'est-à-dire contenant une proportion d'oxygène supérieure à celle de l'air ambiant, on assiste à un vieillissement encore plus rapide de ces composants, en particulier des roulements, car l'oxygène présent dans le flux de gaz de fuite peut alors venir oxyder les lubrifiants et diminuer ainsi leur efficacité, donc engendrer une usure encore plus rapide de ces composants du moteur.

Au vu de cela, le problème qui se pose est de proposer une micro-soufflante améliorée ne présentant pas les problèmes d'étanchéité au niveau de l'arbre moteur susmentionnés, permettant de rendre le circuit de gaz complètement étanche et donc d'éviter que du gaz de fuite ne puisse détériorer les roulements du moteur et conduire à une usure prématuré de ce dernier.

La solution de l'invention est alors une micro-soufflante comprenant moteur électrique comprenant un corps de moteur, c'est-à-dire définissant, un compartiment-moteur, et un arbre-moteur rotatif de forme allongée, ledit arbre-moteur étant maintenu ou guidé par au moins un dispositif à roulements agencé dans ledit corps de moteur, ledit arbre-moteur traversant la paroi supérieure du corps de moteur par un orifice aménagé dans ladite paroi supérieure et faisant saillie à l'extérieur dudit corps de moteur, caractérisée en ce qu'une pièce d'étanchéité traversée par l'arbre-moteur est agencée au niveau de l'orifice.

Selon le cas, la micro-soufflante de l'invention peut comprendre l'une ou plusieurs des caractéristiques techniques suivantes :
- le corps de moteur renferme et maintient les roulements, les bobines et l'arbre-rotatif.
- l'arbre-rotatif fait saillie hors du corps du moteur au travers d'un couvercle amovible formant la paroi supérieure du corps de moteur.
- la pièce d'étanchéité est maintenue en position par un dispositif de maintien.
- le dispositif de maintien est une bride ou analogue.
- la pièce d'étanchéité est positionnée au contact de la paroi supérieure du corps de moteur.
- la pièce d'étanchéité (6) est formée d'un matériau déformable apte à assurer une étanchéité fluidique, de préférence le matériau est du polytétrapluoroéthylène (PTFE) ou polyéthylène (PE).
- la pièce d'étanchéité est une pièce de révolution.
- la pièce d'étanchéité est maintenue en position fixe par rapport à l'arbre-moteur.
- la pièce d'étanchéité est traversée par un passage interne ayant un diamètre interne sensiblement égal au diamètre externe de l'arbre-moteur, de préférence l'arbre-moteur rotatif a une forme allongée tubulaire.
- l'arbre-moteur porte une roue à ailettes, la pièce d'étanchéité étant agencée entre la surface supérieure du corps de moteur et la face inférieure de la roue à ailettes.
- la roue à ailettes est en matériau léger, de préférence en polymère, par exemple en plastique moulé.
- la pièce d'étanchéité est agencée dans un logement aménagé dans la paroi supérieure du corps de moteur, l'orifice débouchant dans le fond dudit logement.
- la pièce d'étanchéité est un joint tournant.
- la roue à ailettes est fixée à l'arbre-moteur de manière à pouvoir être entraînée en rotation lors des rotations dudit arbre-moteur, ledit arbre-moteur étant lui-même entraîné en rotation par le moteur.
- l'arbre-moteur est de forme allongée, notamment de forme tubulaire, par exemple cylindrique.
- la roue à ailettes est agencée dans un compartiment à roue délimité par une volute supérieure et une volute inférieure, fixées l'une à l'autre.
- la volute supérieure comprend un passage d'entrée de gaz en communication fluidique avec le compartiment à roue contenant la roue à ailettes. Ce passage d'entrée de gaz permet une entrée de gaz, typiquement d'air ou d'air enrichi en oxygène, au sein compartiment à roue, ledit gaz étant aspiré par la roue à ailettes, lors de ses rotations.
- la volute supérieure et la volute inférieure forment entre elles un conduit de sortie ou d'évacuation de gaz permettant d'extraire le gaz aspiré par la roue à ailettes, du compartiment à roue.
- la volute supérieure est surmontée d'un pavillon comprenant une ouverture en communication fluidique avec le passage d'entrée de gaz de la volute supérieure.

L'invention concerne en outre un appareil d'assistance respiratoire équipé d'une micro-soufflante selon l'invention, telle que décrite ci-avant et illustrée ci-après.

De préférence, l'appareil d'assistance respiratoire comprend une sortie de gaz à laquelle peut être raccordé fluidiquement un conduit d'acheminement de gaz, tel qu'un tuyau flexible, permettant de convoyer le gaz délivré par la turbine de l'appareil jusqu'à une interface-patient, tel qu'un masque respiratoire ou analogue, raccordé fluidiquement au conduit d'acheminement de gaz.

L'invention va maintenant être mieux comprise grâce à la description détaillée suivante faite en références aux Figures annexées parmi lesquelles :
- la Figure 1 est une vue de 3/4 d'une partie d'une micro-soufflante selon la présente invention,
- la Figure 2 montre la micro-soufflante de la Figure 1 équipée d'une roue à ailettes,
- les Figures 3 et 4 sont des représentations de la micro-soufflante de la Figure 2 en vue latérale et en vue latérale en coupe, respectivement,
- la Figure 5 est un schéma en coupe de la micro-soufflante de la Figure 4,
- la Figure 6 est une vue grossie d'une partie de la Figure 5,
- la Figure 7 représente une vue en coupe d'une micro-soufflante d'architecture classique,
- la Figure 8 est une vue grossie des roulements de la micro-soufflante de la Figure 7,
- la Figure 9 représente une vue légèrement de dessus d'une micro-soufflante selon l'invention équipée de ses volutes, et
- la Figure 10 est une vue latérale de la micro-soufflante de la Figure 9.

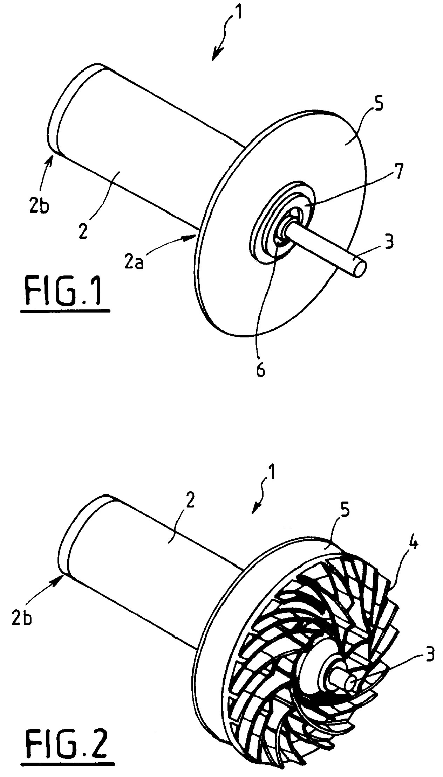

La Figure 1 représente un mode de réalisation, en vue de 3/4, d'une partie d'une micro-soufflante 1 conforme à la présente invention non équipée de la roue à ailettes, ni de ses volutes.

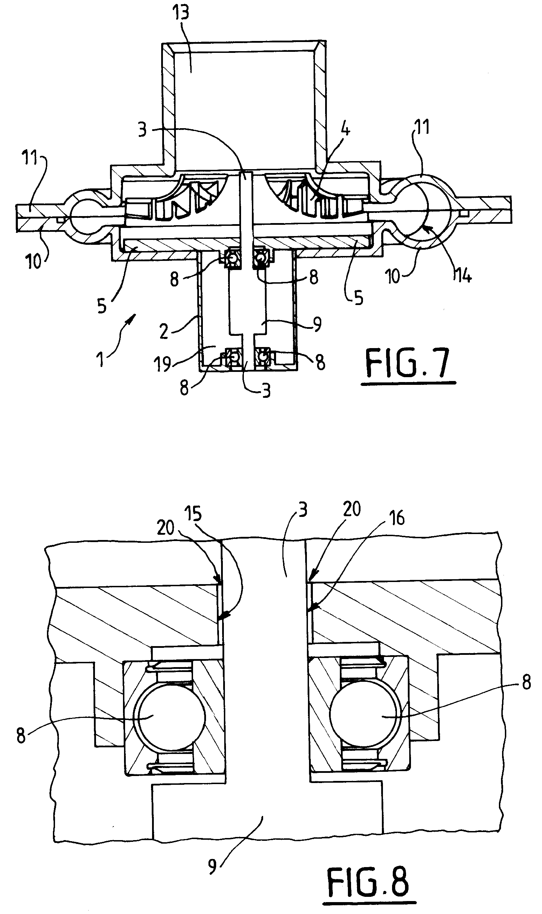

Cette micro-soufflante 1 a globalement la structure d'une micro-soufflante 1 classique, comme par exemple celle illustrée sur les Figures 7 et 8.

Plus précisément, elle comprend un moteur 9 (visible sur Fig. 7) alimenté en courant électrique et délimité par un corps de moteur 2 ou boitier, ici de forme cylindrique, définissant un compartiment ou volume interne, encore appelé compartiment-moteur 19, renfermant un arbre rotatif, des bobines/aimants et les autres éléments constitutifs du moteur 9. Le moteur 9 est piloté via des câbles électriques (non montrés) ou analogues venant se raccorder à l'extrémité inférieure 2b du corps de moteur 2.

Le corps de moteur2 comprend à son extrémité supérieure 2a, un flasque avant 5 en forme de disque auquel vient de fixer la volute inférieure 10, comme illustré sur les Figures 7 et 8. Le flasque avant 5 constitue donc tout ou partie de la paroi ou face supérieure du corps de moteur 2.

L'arbre ou axe 3 rotatif du moteur 9 agencé dans le corps de moteur2 fait saillie hors dudit corps de moteur2 à son extrémité supérieure 2a en traversant un orifice 16 aménagé dans la face supérieure du corps de moteur 2, par exemple au travers du flasque 5 dans le mode de réalisation illustré en Figures 7 et 8 dans lequel la paroi supérieure du corps de moteur 2 est constituée par le flasque 5 qui forme une sorte de couvercle à l'extrémité supérieure 2a du corps de moteur2.

Comme visible sur la Figure 2, cet arbre 3 porte une roue à ailettes 4 et l'entraîne en rotation, pendant le fonctionnement du moteur 9, ce qui permet d'aspirer de l'air au sein du compartiment 12 dans lequel est agencée la roue 4, lequel est formé entre les volutes supérieure 11 et inférieure 10.

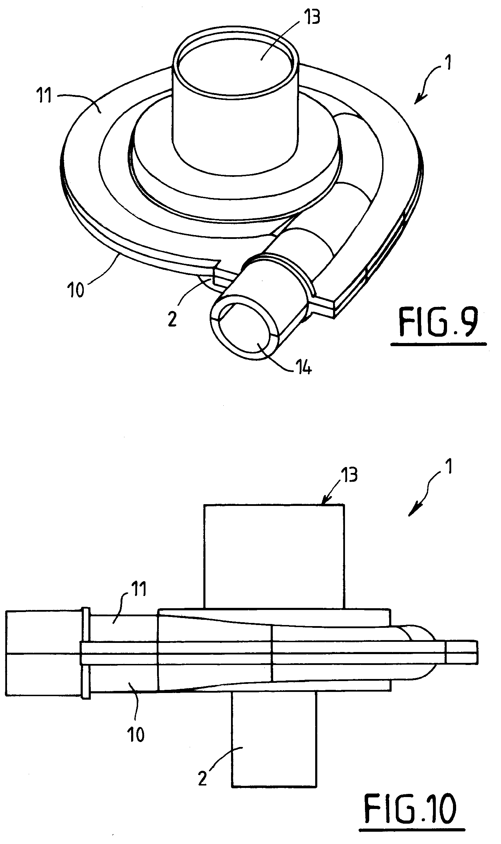

Les Figures 9 et 10 montrent la micro-soufflante 1 de la Figure 2 équipée de ses volutes 10, 11. Les volutes 10, 11 surmontent le corps de moteur 2 du moteur 9. La volute inférieure 10 vient prendre appui sur la surface inférieure 5b du flasque 5, comme montré en Figure 7.

L'air pénètre dans le compartiment interne 12 défini par les volutes 10, 11 par l'ouverture 13 aménagée dans la volute supérieure 11. L'air aspiré par la roue à ailettes 4, lors de ses rotations, est expulsée du compartiment 12 interne par un conduit de sortie 14 permettant de véhiculer l'air qui est ensuite envoyé vers le patient, via un conduit flexible ou analogue.

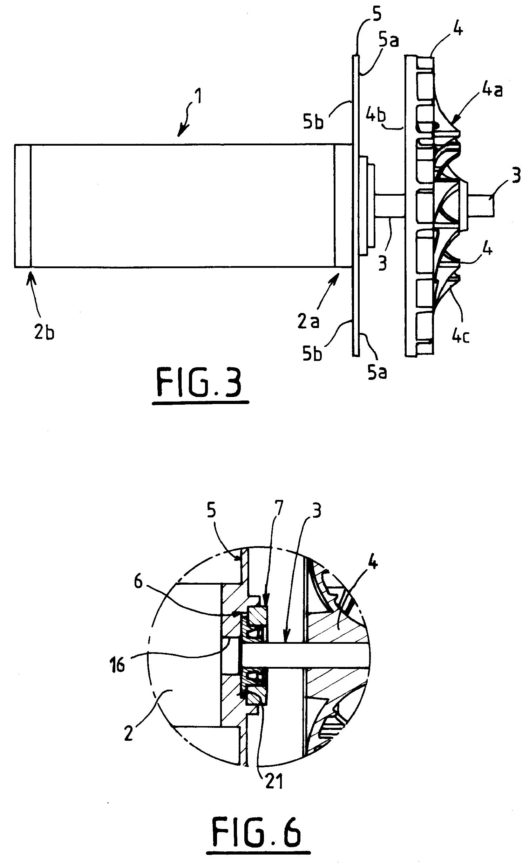

Comme visible sur les Figures 3, 4 et 7, la face inférieure 4b de la roue 4 à ailettes est espacée et sensiblement parallèle à la surface supérieure 5a du flasque avant 5 en forme de disque, alors que la face supérieure 4a de la roue 4 porte les ailettes 4c. En général, la roue 4 est faite en matériau léger, typiquement en polymère.

Habituellement, l'arbre-moteur 3 est maintenu et/ou guidé, lors de ses rotations, par des dispositifs à roulements 8 lubrifiés par des lubrifiants de type huile ou analogue. De préférence, des dispositifs à roulements 8 sont agencés de part et d'autre du moteur 9, autour de l'axe 3, comme visible sur les Figures 7 et 8.

Le problème qui se pose est qu'il existe toujours un jeu 20, c'est-à-dire un espacement, entre l'axe 3 et la paroi interne 15 de l'orifice 16 traversant la face supérieure du corps de moteur2, par lequel l'axe 3 sort du corps de moteur 2 pour permettre une rotation de l'axe 3 dans l'orifice 16. Ce jeu 20, bien que faible, est suffisant pour qu'il se produise des passages intempestifs de gaz sous pression depuis le compartiment 12 vers l'intérieur du corps de moteur 2.

Or, comme déjà expliqué, ces entrées d'air sous pression engendrent une usure prématurée des roulements 8 du moteur 9 car la pression gazeuse chasse le (ou les) lubrifiant qui est supposé limiter l'usure des roulements 8 et l'oxygène qui y est présent, en particulier lorsqu'il s'agit d'air enrichi en oxygène (>22% vol. O₂), détériore ledit lubrifiant en l'oxydant.

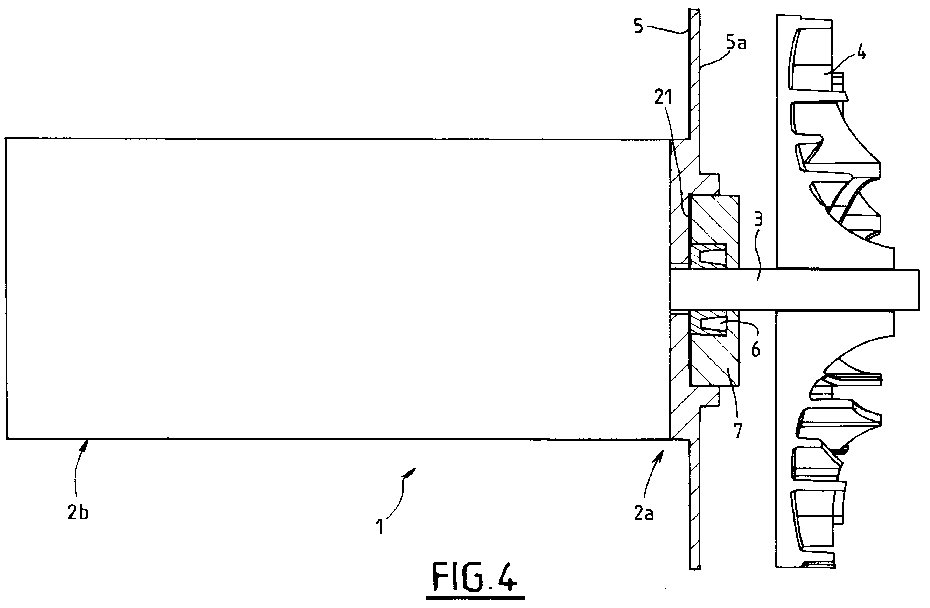

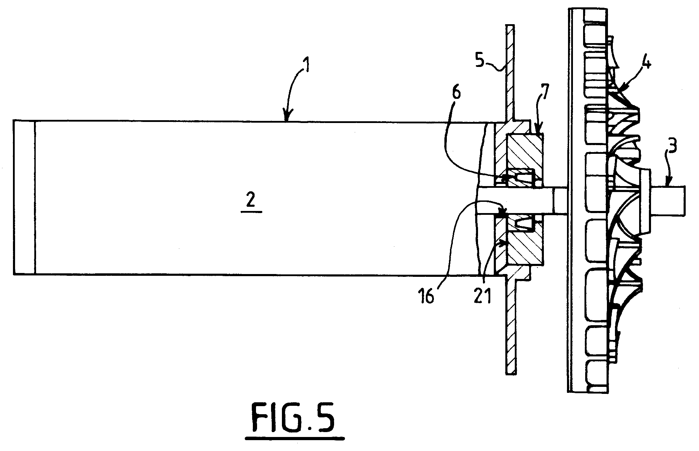

Pour palier ce problème, conformément à la présente invention et comme illustré sur les Figures 4-6, on réalise une étanchéité gazeuse complète ou quasi-complète au niveau de l'arbre-moteur 3 grâce à l'agencement autour dudit arbre 3, au niveau de l'extrémité supérieure du corps de moteur2, d'une pièce d'étanchéité 6, qui peut être par exemple un joint tournant.

Cette pièce d'étanchéité 6 est traversée par l'arbre-moteur 3 et est retenue ou fixée au corps de moteur 2 par un dispositif de maintien 7 adapté, par exemple une bride. Elle est formée d'un matériau déformable, par exemple un polymère, typiquement un polymère de type PE ou PTFE.

Avantageusement, la pièce d'étanchéité 6 est agencée dans un logement 21 aménagé dans la paroi supérieure 5 du corps de moteur 2, l'orifice 16 par lequel passe l'arbre 3 débouchant dans le fond dudit logement 21. Elle y est retenue par la bride ou analogue.

La pièce d'étanchéité 6 constitue dès lors une barrière mécanique étanche au gaz qui permet de protéger certains éléments du moteur contenus dans le corps de moteur 2, en particulier les roulements, en évitant que :
- d'une part, le différentiel de pression existant entre l'intérieur du corps de moteur 2 et intérieur du compartiment à roue 4 ne conduise à des entrées intempestives de gaz sous pression dans le corps de moteur 2 qui viendraient alors opérer un balayage gazeux des roulements et chasser le lubrifiant qui protège ces roulements ; et
- d'autre part, de l'oxygène ne puisse pénétrer dans le corps de moteur 2 et ne vienne oxyder et donc détériorer le lubrifiant protégeant les roulements.

Plus précisément, cette pièce d'étanchéité 6 vient se fixer en amont de l'orifice 16 traversé par l'axe 3 et assurer ainsi une étanchéité maximale au niveau de l'axe 3 et de la surface supérieure du corps de moteur 2, donc constituer une barrière mécanique étanche s'opposant au passage de gaz dans le jeu 20 existant entre la paroi interne 15 dudit orifice 16 et l'axe 3.

Ainsi, le flux de gaz qui balayera les roulements 8 sera nul ou extrêmement réduit et la quantité d'oxygène susceptible d'oxyder le lubrifiant sera également très réduite, voire nulle. La durée de vie de la micro-soufflante en sera dès lors améliorée.

D'une façon générale, l'appareil de l'invention équipé de la micro-soufflante décrite ci-avant peut être utilisé pour traiter des pathologies respiratoires de tout type, en particulier apnée du sommeil, broncho-pneumopathie chronique obstructive (BPCO), troubles liés à l'obésité...

## Revendications

1. Micro-soufflante (1) pour appareil d'assistance respiratoire comprenant un moteur électrique (9) comprenant un corps de moteur (2) et un arbre-moteur (3) rotatif de forme allongée, ledit arbre-moteur (3) étant maintenu ou guidé par au moins un dispositif à roulements (8) agencé dans ledit corps de moteur (2), ledit arbre-moteur (3) traversant la paroi supérieure (5) du corps de moteur (2) par un orifice (16) aménagé dans ladite paroi supérieure (5) et faisant saillie à l'extérieur dudit corps de moteur (2), **caractérisé en ce qu'**une pièce d'étanchéité (6) traversée par l'arbre-moteur (3) est agencée au niveau de l'orifice (16).

2. Micro-soufflante selon la revendication précédente, **caractérisée en ce que** la pièce d'étanchéité (6) est maintenue en position par un dispositif de maintien (7).

3. Micro-soufflante selon l'une des revendications précédentes, **caractérisée en ce que** le dispositif de maintien (7) est une bride.

4. Micro-soufflante selon l'une des revendications précédentes, **caractérisée en ce que** la pièce d'étanchéité (6) est positionnée au contact de la paroi supérieure (5) du corps de moteur (2).

5. Micro-soufflante selon l'une des revendications précédentes, **caractérisée en ce que** la pièce d'étanchéité (6) est formée d'un matériau déformable apte à assurer une étanchéité fluidique choisi parmi le polytétrapluoroéthylène (PTFE) et le polyéthylène (PE).

6. Micro-soufflante selon l'une des revendications précédentes, **caractérisée en ce que** la pièce d'étanchéité (6) est une pièce de révolution.

7. Micro-soufflante selon l'une des revendications précédentes, **caractérisée en ce que** la pièce d'étanchéité (6) est maintenue en position fixe par rapport à l'arbre-moteur (3).

8. Micro-soufflante selon l'une des revendications précédentes, **caractérisée en ce que** la pièce d'étanchéité (6) est traversée par un passage interne ayant un diamètre interne sensiblement égal au diamètre externe de l'arbre-moteur (3).

9. Micro-soufflante selon l'une des revendications précédentes, **caractérisée en ce que** l'arbre-moteur (3) rotatif a une forme allongée tubulaire.

10. Micro-soufflante selon l'une des revendications précédentes, **caractérisée en ce que** l'arbre-moteur (3) porte une roue (4) à ailettes, la pièce d'étanchéité (6) étant agencée entre la surface supérieure (5) du corps de moteur (2) et la face inférieure (4b) de la roue (4) à ailettes.

11. Micro-soufflante selon l'une des revendications précédentes, **caractérisée en ce que** la pièce d'étanchéité (6) est agencée dans un logement (21) aménagé dans la paroi supérieure (5) du corps de moteur (2), l'orifice (16) débouchant dans le fond dudit logement (21).

12. Micro-soufflante selon l'une des revendications précédentes, **caractérisée en ce que** la pièce d'étanchéité (6) est un joint tournant.

13. Appareil d'assistance respiratoire équipé d'une micro-soufflante (1) selon l'une des revendications précédentes.
